# EUROPEAN PATENT SPECIFICATION

(11) **EP 3 311 774 B1**
(45) Date of publication and mention of the grant of the patent: **11.05.2022**
(21) Application number: 16194649.6
(22) Date of filing: 19.10.2016
(51) Int. Cl.: A61F 2/24

(54) **SELF-EXPANDABLE ATRIOVENTRICULAR VALVE AND SYSTEM OF CARDIAC VALVES**
SELBSTEXPANDIERENDE ATRIOVENTRIKULÄRE KLAPPE UND SYSTEM FÜR HERZKLAPPEN
VALVULE ARTÉRIOVENTRICULAIRE AUTO-EXTENSIBLE ET SYSTÈME DE VALVULES CARDIAQUES

(43) Date of publication of application: 25.04.2018
(73) Proprietor: P+F Products + Features Vertriebs GmbH, 1190 Wien (AT)
(72) Inventor: Kiss, Katharina, 1190 Vienna (AT); Agreli, Guilherme, Sao Paulo State (BR)
(74) Representative: Manitz Finsterwald Patent- und Rechtsanwaltspartnerschaft mbB

(56) References cited:
- EP-A1- 2 641 569
- EP-A1- 2 732 796
- WO-A1-2011/057087
- WO-A1-2014/209232
- WO-A1-2016/073741
- CA-A1- 2 950 764
- US-A1- 2013 261 738
- US-A1- 2016 228 251

## Description

The present invention relates to self-expandable atrioventricular valves, such as a replacement mitral valve or a replacement tricuspid valve and generally to the field of devices used to treat mitral valve disease by minimally invasive methods, without use of extracorporeal circulation or circulatory arrest. The invention further relates to a system of cardiac valves.

A healthy heart facilitates oxygenated blood flow to the extremities. The heart is comprised of four chambers: the right and left sides, which manage deoxygenated and oxygenated blood respectively. Deoxygenated blood from the upper and the lower extremities, travels through the caval veins into the right atrium. It is pumped through the tricuspid valve and into the right ventricle. During systole, blood is pumped from the right ventricle, through the pulmonary valve and into the lungs. Following oxygenation, blood is pumped back to the left side of the heart through the pulmonary vein. Oxygenated blood flows through the mitral valve into the left ventricle. During systole, the left ventricle ejects the blood through the aortic valve to the rest of the body.

Proper opening and closing of the atrioventricular valves is dependent on the function of all of the structures involved with leaflet function, specifically the annulus, the leaflets, the chordae tendinae, the papillary muscles and a healthy myocardial wall. During ventricular filling (diastole), the atrioventricular valves remain open and during ventricular contraction (systole), the valves close as a consequence of complete leaflet apposition. In instances where complete apposition of the leaflets is not achieved, leakage or regurgitation across the valve affects cardiac performance and health. Severe regurgitation or leakage can lead to hemodynamic deterioration and/or heart failure.

Moderate or higher tricuspid valve regurgitation affects cardiac functional performance, and is typically indicative of a broader primary disease such as left heart dysfunction. Primary dysfunction can include pulmonary hypertension, right ventricular volume overload and right ventricular disease. Similarly, mitral regurgitation or leakage is primarily due to degenerative valve disease. The presence of dysfunctional support structures also contributes to mitral regurgitation, specifically as a consequence of a heart attack (ischemic mitral regurgitation) or ventricular dilation (functional mitral regurgitation). Other causes of atrioventricular regurgitation include acquired conditions (i.e. endocarditis, rheumatic heart disease) and congenital anomalies.

Atrioventricular regurgitation also impact the caval veins leading to the atrium. During tricuspid regurgitation, pressure and flow changes in the right atrium causes the inferior vena cava (IVC) and the superior vena cava (SVC) to dilate. Additionally, veins feeding the IVC and the SVC are also impacted by these effects. Specifically, tricuspid regurgitation causes the azygos and the hepatic veins, which are located cranial and caudal to the right atrial opening respectively, to dilate. This leads to overall hemodynamic deterioration and liver dysfunction. Physical symptoms of tricuspid regurgitation include fatigue, loss of appetite and abdominal fullness. Similarly, mitral regurgitation impacts the pulmonary vein and can cause fluid buildup in the lungs. Chronic mitral regurgitation often leads to left ventricular remodeling or dilation of the left ventricle. Physical symptoms of mitral regurgitation include fatigue, decreased exercise tolerance, shortness of breath, and swollen feet. If left untreated, either atrioventricular severe regurgitation can lead to pulmonary hypertension, atrial fibrillation or heart failure.

On use of transcatheter valves to treat e.g. mitral disease, these modify the anatomy of the structures surrounding the mitral valve, deforming the aortic valve or lead to an occlusion of the coronaries arteries or veins. A deformation of anatomical structures can lead to a malfunction of the heart, including the aortic valve. Other complications brought about by an implantation of a replacement mitral valve is a change in the flow properties of the blood exiting the mitral valve due to the design of the replacement valve or thrombus formation in the atrial portion respectively. Moreover, if the atrioventricular valve is not placed correctly, this cannot seal the ventricles from the atria correctly such that the underlying pathology is not treated correctly, creating a paravalvular leakage.

Priort art valves are disclosed in the following documents WO2011/057087A1; WO2014/209232A1; US2016/228251A1; WO2016/073741A1; CA2950764A1; EP2641569A1; US2013/261738A1; and EP2732796A1.

For this reason it is an object of the present invention to provide a medical implant or prosthesis that can provide relief from tricuspid valve leakage or regurgitation and from mitral valve leakage or regurgitation. It is a further object of the present invention to minimize the deformation of anatomical structures and to ensure simple and reliable placement at the native valve in question. It is yet a further object of the present invention to minimize the recovery time of a patient following the treatment with such a medical implant or prosthesis.

These objects are satisfied by a self-expandable atrioventricular valve having the features in accordance with claim 1.

Such a self-expandable atrioventricular valve that is expandable from an un-deployed state into a deployed state and that in the deployed state comprises:
- a stent frame forming a body, with the body having a height extending from an inlet to an outlet of the self-expandable atrioventricular valve in an axial direction and with the body circumferentially extending in a circumferential direction around the axial direction,
- a unidirectional valve member arranged within the body in the region of the outlet,
- a plurality of fingers that is arranged in the region of, preferably directly at, the inlet, with the plurality of fingers projecting radially outwardly from the body in a radial direction and with the plurality of fingers forming at least part of, preferably a complete, upper end of the self-expandable atrioventricular valve together with the inlet, and
- a plurality of anchors that is arranged in the region of the outlet, with a first end of each of the plurality of anchors projecting radially outwardly from the body in the radial direction in the region of the outlet and with the first ends of the plurality of anchors forming at least part of, preferably a complete, lower end of the self-expandable atrioventricular valve together with the outlet.

The provision of a self-expandable atrioventricular valve permits the use of minimally invasive techniques reducing the recovery time of a patient following surgery.

In this connection it should be noted that the lower end of the self-expandable atrioventricular valve can only be formed by the plurality of anchors and the outlet. It should further be noted that the upper end of the self-expandable atrioventricular valve can only be formed by the plurality of fingers and the inlet. In this way on deployment of the replacement valve structure the lower and upper end can be used to clamp the native atrial wall in order to produce a replacement valve that is adapted to an e.g. native mitral valve and no components of the atrioventricular valve project into the respective ventricle.

By providing a stent frame that has no structures that project beyond the upper end or the lower end respectively into the flow path of the blood pumped through the respective valve during diastole, a distortion or alteration of the blood flow pattern is avoided.

Moreover, the replacement valve is significantly shorter than prior art stents in which the valve member is typically arranged at the center of the stent frame and not in the region of the outlet and in which structures of the stent frame thus also project into the space of the respective ventricle. This is presently avoided by forming the outlet and the inlet of the valve as part of the respective upper and lower ends of the atrioventricular valve.

In this connection it should be noted that it is preferred if tips of leaflets forming the valve member lie in a plane comprising the outlet and the plurality of fingers, i.e. in a plane at least essentially perpendicular to a flow of blood from the inlet through the mitral valve via the outlet into the left ventricle.

Preferably the plurality of anchors forms an atrial connection face and the plurality of fingers forms a ventricular connection face such that in use the mitral valve can clamp an atrial wall between the atrial connection face and the ventricular connection face so that a short length mitral valve results that does not project beyond the opening of the native mitral valve. In this way the flow properties of the blood exiting the mitral valve are not altered considerably.

It is preferred if the stent frame at least substantially has the shape of an inner bearing race, thereby an as short as possible stent frame can be made available.

Advantageously a height of the body is selected in the range of 0.3 to 2.0 cm between the outlet and the inlet. These dimensions reflect a thickness of the native atrial wall.

It is preferred if an overall height of the atrioventricular valve between a lowest point of the lower end, i.e. the inlet, and an uppermost point of the atrioventricular valve, i.e. the plurality of fingers, is selected in the range of 0.3 to 2.5 cm. In this way particularly short atrioventricular valves are made available that are similar in geometry to the native geometry and that do not alter the flow of blood through the valve into the respective ventricle.

In this way the height of the atrioventricular valve is selected such that it accommodates the unidirectional valve member within its body with at least one end of the unidirectional valve member ending at the oulet.

Preferably the plurality of anchors that form the lower end radially project away from the outlet towards the upper end. Such anchors can maintain a position of the native leaflets and thereby also enhance the function of a replacement atrioventricular valve.

Preferably the plurality of fingers and the plurality of anchors are arranged such that they at least generally project in the same axial direction. In this way the atrioventricular valve is adapted to the native shape of the atrial wall separating e.g. the left atrium from the left ventricle of a mammalian heart.

It should be noted in this connection that a cross-section of the lower end in the axial direction can have the shape of a truncated cone.

Advantageously a second end of the plurality of anchors extends over a height of the body. Thereby the body of the stent frame is reinforced in the regions having the second ends of the plurality of anchors.

At least some of the plurality of anchors is configured as respective commissures for the valve member that is longitudinally attached to the plurality of anchors, preferably to the second ends thereof forming the commissures, and over the height of the body and circumferentially within the body in the region of the inlet, so that an upper surface of the valve member is present in the region of the inlet and preferably forms part of the upper end.

These commissures are then present in the reinforced regions of the stent frame enabling a long lifetime of such a replacement valve. Moreover, by attaching the valve member in this way leaks between the valve member and the body can be avoided.

Preferably the valve member comprises at least two leaflets, and in particular comprises four leaflets. It has hitherto been found that the use of four leaflets in an atrioventricular valve of elliptical design leads to a surgical valve that performs like a well-functioning native valve.

The atrioventricular valve further comprises a plurality of elongate struts that form at least a part of the body of the stent frame, wherein the plurality of elongate struts, the plurality of anchors and the plurality of fingers are formed from the same material, such as nitinol. In this way a one-piece stent frame can be provided that is made from a flexible self-expanding material.

The plurality of elongate struts, the plurality of anchors and the plurality of fingers have at least approximately the same thickness in the radial direction, and with the plurality of anchors and the plurality of fingers respectively having a greater width than the plurality of elongate struts in a circumferential direction of the body.

It is preferred if the plurality of fingers form a part of an inlet flange of the self-expandable atrioventricular valve forming the upper end. Likewise it is advantageous if the plurality of anchors form a part of an outlet flange of the self-expandable atrioventricular valve forming the lower end. The use of flanges as an upper and lower end can prevent the formation of leaks between the atrial and ventricular portion of the heart in the region of the replacement valve, as the flanges can respectively form a seal that seals off the upper and lower ends. Moreover, the inlet flange can form the atrial connection face and the outlet flange can form the ventricular connection face such that in use the mitral valve can clamp an atrial wall between the atrial connection face and the ventricular connection face. Preferably the plurality of fingers and the plurality of anchors - and hence also the outlet and inlet flanges - act as cooperating clamps of the self-expandable atrioventricular valve. In this way cardiac tissue can be clamped between the plurality of fingers and the plurality of anchors to ensure a tight fit of the replacement atrioventricular valve.

Advantageously the plurality of fingers is formed by or extends from at least some of the plurality of elongate struts. Thereby a particularly simple design of the stent frame can be achieved.

Preferably at least some of the plurality of fingers comprises eyelets for a fixation of the self-expandable atrioventricular valve. Likewise at least some of the plurality of anchors can comprise openings or apertures for a fixation of the self-expandable atrioventricular valve.

It is preferred if the plurality of anchors is formed by or extends from at least some of the material forming the body. Likewise the plurality of anchors can comprise a first group of anchors and a second group of anchors, with a respective anchor of the first group of anchors being shorter than a respective anchor of the second group of anchors. Similarly the plurality of fingers can be formed by or extend from at least some of the material forming the body and can also comprise a first group of fingers and a second group of fingers, with a respective finger of the first group of fingers being shorter than a respective finger of the second group of fingers.

It should be noted in this connection that at least some of the fingers of one of the groups of fingers can comprise eyelets and/or that at least some of the anchors of one of the groups of anchors can comprise openings, whereas the respective other group of fingers or anchors respectively can include no eyelets or no openings.

It is preferred if the body has an at least generally elliptical shape in a cross-section perpendicular to the axial direction. The selection of an elliptical shape means that the implant is configured similar to the native mitral valve and thereby avoids a deformation of the native anatomy and thereby does not influence the heart in an unpredictable manner.

It is even more preferable if the body has an at least generally cylindrical outer shape over its height in the axial direction. This enables a smooth fit of the body to the native valve.

Preferably a diameter of the body at the inlet is greater than or approximately the same as a diameter of the body at the outlet. In this way the mitral valve can be designed in accordance with the anatomical shape of the mammalian heart.

It is preferred if the self-expandable atrioventricular valve further comprises anti-leakage material, with the anti-leakage material being arranged to cover at least part of an inner and/or an outer surface of the body, preferably with the anti-leakage material being arranged to at least substantially completely cover the inner and/or the outer surface of the body, optionally wherein the anti-leakage material at least partly covers the plurality of fingers that form the upper end and/or the plurality of anchors that form the lower end.

In this way unwanted leaks between e.g. the left atrium and the left ventricle can be avoided ensuring an improved function of the replacement mitral valve.

In a further aspect the present invention relates to a system of cardiac valves comprising a self-expandable atrioventricular valve, preferably as discussed herein and a surgical valve for replacement of one of the aortic valve and the pulmonary valve.

The advantages discussed in the foregoing with respect to the atrioventricular valve likewise hold true with respect to the system of cardiac valves.

In a further aspect the present invention relates to a method of delivering and deploying a self-expandable atrioventricular valve in accordance with the invention, preferably by means of minimally invasive surgery using a delivery device. The method comprises the steps of:
- inserting the self-expandable atrioventricular valve into one of the atria;
- placing the self-expandable atrioventricular valve at the position of the natural atrioventricular valve;
- expanding the self-expandable atrioventricular valve at the position of the native atrioventricular valve;
- anchoring the outlet of the self-expandable atrioventricular valve to the cardiac tissue by means of the plurality of anchors;
- attaching the self-expandable atrioventricular valve to the cardiac tissue by means of at least one of an interference fit, sutures, staples, adhesively bonding, clamps and clips; and
- clamping cardiac tissue between the plurality of anchors and the plurality of fingers at the position of the natural atrioventricular valve.

The invention will be described in detail by means of embodiments and with reference to the drawings. These show preferred deployment locations and embodiments of the valve. The features described may be configured in various combinations, which are encompassed in this document. The drawings show:
- Fig. 1: a side view of a first embodiment of a mitral valve having a first kind of stent frame;
- Fig. 2: a top view of the first embodiment of Fig. 1;
- Fig. 3: a photograph of a further embodiment of a mitral valve arranged within a heart;
- Fig. 4: a delivery device for deploying the mitral valve of Fig. 1 in a heart;
- Fig. 5: a further kind of stent frame;
- Fig. 6a & 6b: schematic views of a further kind of stent frame;
- Fig. 7a & 7b: schematic views of a further kind of stent frame;
- Fig. 8a & 8b: schematic views of a further kind of stent frame;
- Fig. 9a & 9b: schematic views of a further kind of stent frame; and
- Fig. 10: schematically, a system of cardiac valves installed in a mammalian heart.

The following detailed description is merely exemplary in nature and is not intended to limit the invention or the application and uses of the invention. Although the description of embodiments hereof is in the context of mitral heart valve replacement, the invention may be adapted to be used for other valve replacement such as in tricuspid valve replacement. Furthermore, any expression relating to direction or position in this application is made relative to the position of installation and/or to the position in the Figures.

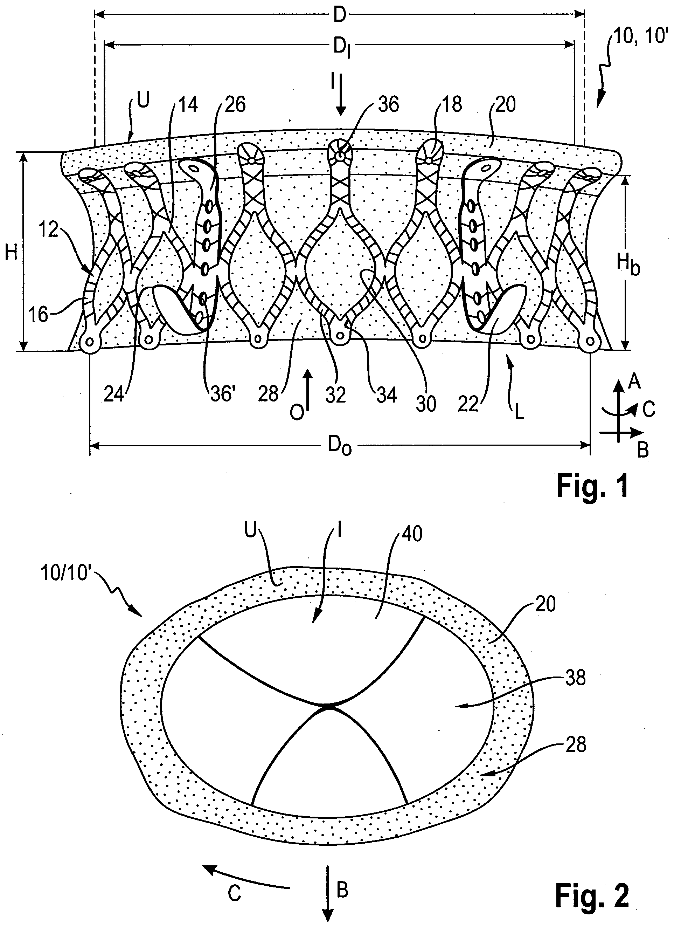

Fig. 1 shows a side view of a first embodiment of a self-expandable atrioventricular valve 10 configured as a mitral valve 10'. The mitral valve 10' is present in a deployed state, this means in an expanded state. The mitral valve 10' comprises a stent frame 12 formed from a plurality of elongate struts 14. A part of each of the plurality of elongate struts 14 forms a body 16 of the mitral valve 10'. A height H_{b} of the body 16 extends from an inlet I to an outlet O in an axial direction A.

A plurality of fingers 18 is arranged at the inlet I and forms an upper end U of the self-expandable atrioventricular valve 10 together with the inlet. Some of the plurality of fingers 18 is formed as extensions of the plurality of elongate struts 14 in the example shown in Fig. 1. The plurality of fingers 18 is radially deflected outwardly from the body 16 in the radial direction B directly at the inlet I. The plurality of fingers 18 thereby forms a part of an inlet flange 20 of the self-expandable atrioventricular valve 10 forming part of the upper end U of the mitral valve 10' together with the inlet I.

Furthermore, a plurality of anchors 22 is provided at the mitral valve 10'. A first end 24 of each of the plurality of anchors 22 projects radially outwardly from the body 16 in the region of the outlet O and forms a lower end L of the mitral valve 10' together with the outlet O. The first ends 24 of the plurality of anchors 22 also extends towards the upper end U.

The first ends 24 of the plurality of anchors 22 are formed by a flexible frame and are provided to capture leaflets of e.g. a native mitral valve and to keep the position of these natural leaflets close to the mitral valve 10' described herein.

A second end 26 of the plurality of anchors 22 extends in the axial direction A towards the inlet I along the height H_{b} of the body 16 in the form of bridges.

Typical heights H_{b} of such bodies 16 of such mitral valves 10' are selected in the range of 0.3 to 2 cm. In the present instance the body has a height H_{b} of 1.5 cm. The mitral valve 10' typically has an overall height H of 0.3 to 2.5 cm measured from the outlet O or lower end L respectively, i.e. the lowest point of the mitral valve 10', to an uppermost point of the mitral valve 10' present at the inlet flange 20. In the present instance the overall height H is 0.7 cm.

The general outer shape of the mitral valve 10' can be compared to that of an inner bearing race. This means that an outer contour of the mitral valve 10' has a region of enlarged outer diameter at the inlet I and defined by the plurality of fingers 18, a region of reduced outer diameter that is defined by an outer diameter of the body 16 and that is arranged between the inlet I and the outlet O and a region of enlarged outer diameter at the outlet O that is defined by the plurality of anchors 22.

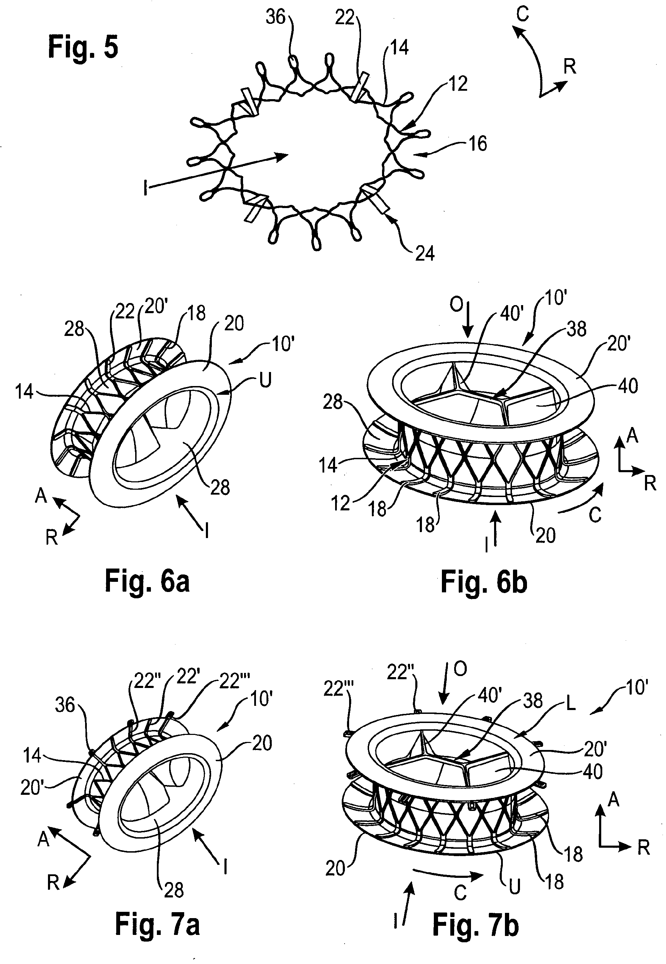

It should be noted in this connection that some of the plurality of fingers 18 are formed as extensions of the plurality of anchors 22 in the example of Fig. 1. It should further be noted in this connection that embodiments can exist in which the plurality of fingers 18 can be formed such that they only form extensions of at least some of the plurality of elongate struts 14 (see e.g. Fig. 5 in this regard).

An inner diameter Dₒ of the body 16 at the outlet O, i.e. the diameter of the outlet, is greater than an inner diameter Dᵢ of the body 16 at the inlet I, i.e. the diameter of the inlet. The body 16 has a generally planar inner surface 30 with an inner diameter D of the body 16 reducing over the height H_{b} of the body 16 from the outlet O to the inlet I.

In this connection it should be noted that the inner diameter Dₒ of the body 16 at the outlet O can also be equal to the inner diameter Dᵢ of the body 16 at the inlet I in such a way that the body 16 has an at least substantially like inner diameter D over the height H of the body 16.

Anti-leakage material 28 is affixed to the inner surface 30 of the body 16 and to an outer surface 32 of the plurality of fingers 18 by means of sutures 34. In order to improve the attachment of the anti-leakage material 28 to the stent frame 12, a plurality of eyelets 36 is provided at the plurality of fingers 18 and at the plurality of elongate struts 14. The bridges or second ends respectively also include apertures 36' therein.

It should be noted in this connection that the anti-leakage material 28 can also be attached to an outer surface of the body 16.

It should also be noted in this connection that the anti-leakage material 28 shown in Figs. 1 and 2 comprises woven material, can however likewise be formed by a dip coating process in which a coating is formed on the stent frame 12 by dipping the stent frame into a bath of coating material, a molding process in which a coating is molded to the stent frame 12 or by a spray process in which a coating of anti-leakage material is sprayed onto the stent frame 12.

The plurality of elongate struts 14, the plurality of fingers 18 and the plurality of anchors 22 are formed from the same flexible material namely nitinol in the present instance. The plurality of elongate struts 14, the plurality of fingers 18 and the plurality of anchors 22, have at least approximately the same thickness in the radial direction. Moreover, the plurality of fingers 18 and the plurality of anchors 22 respectively have a greater width than the plurality of elongate struts in a circumferential direction C of the body 16.

Fig. 2 shows a top view of the first embodiment of Fig. 1 onto the upper end U of the mitral valve 10' having the inlet I formed therein. A unidirectional valve member 38 arranged is within the body 16 at the inlet I. The valve member 38 shown in the present example comprises four leaflets 40. It should be noted in this connection that tips of the leaflets 40 (not shown) lie in the plane of the outlet (also not shown).

In this connection it should be noted that blood flows from the inlet I through the unidirectional valve member 38 of the mitral valve 10' via the outlet O into the left ventricle.

It should be noted in this connection that the four leaflets 40 of the valve member 38 are respectively connected to the anti-leakage material 28 covering the inner surface 30 of the body 16. To this end the four leaflets 40 are circumferentially connected to the anti-leakage material in the region of the inlet. Moreover, the leaflets are also longitudinally attached to the second ends 26 of the plurality of anchors 22 that hence act as commissures.

The leaflets 40 are typically formed by biological tissue. In the present case the leaflets 40 are formed by bovine pericardium, but could likewise be formed by porcine pericardium, bacterial cellulose or another suitable material. The number of leaflets 40 used in an atrioventricular valve 10 depends on the shape of the atrioventricular valve 10, for an atrioventricular valve 10 used as a mitral valve 10', an elliptical shape as currently shown has found to be advantageous. Moreover, elliptically shaped mitral valves 10' work best with four leaflets 40. It has namely been found that such a shape adapts best to the natural shape of a natural mitral valve (not shown). The coaptation (area of interference) and length of the leaflets 40 can be adjusted according to the performance criteria defined during manufacturing of the mitral valve 10'.

In this connection it should be noted that the plurality of anchors 22 are also configured as commissures for the leaflets 40 of the valve member 38. To this end the bridges or second ends 26 respectively include the apertures 36' (see Fig. 1) for sewing the leaflets 40 to the stent frame 12.

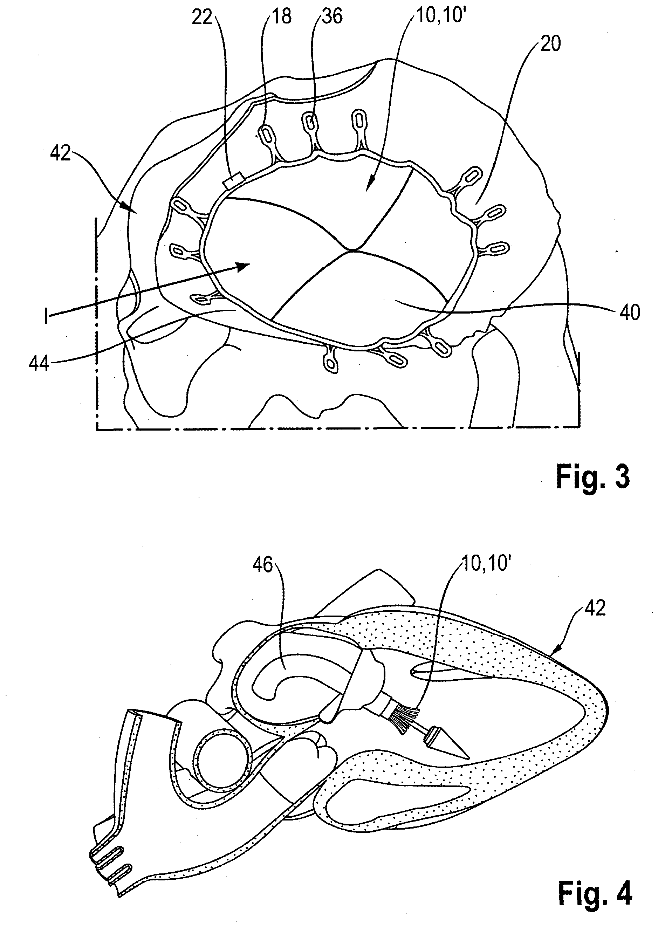

Fig. 3 is a photograph of a further kind of mitral valve 10' placed within a heart 42 as viewed from a left atrium. The body 16 of the mitral valve 10' and the plurality of fingers 18 are adapted to the anatomy of the native mitral valve ring in such a way that the plurality of fingers 18 sit on an atrial wall 44 of the native mitral wall ring, with the body 16 extending through the native mitral valve ring such that the native anatomical structure is deformed as little as possible. Moreover, the mitral valve 10' seals the previously present paravalvular leakage by means of the four leaflets 40, the inlet flange 20 and the anti-leakage material 28 (not shown).

Due to the deflection of the fingers radially outwardly these clamp into position at the atrial wall 44 in the left ventricle. Moreover, no components of the mitral valve 10' project into the space of the left ventricle. Likewise, the plurality of anchors 22 clamp the atrial wall 44 on the atrial side of the native mitral valve ring valve in such a way that the plurality of fingers 18 and the plurality of anchors 22 act as cooperating clamps in the deployed state of the self-expandable atrioventricular valve.

Fig.4 shows a delivery device 46 for deploying the mitral valve 10' into the heart 42. In order to effect this the mitral valve 10' has distal connectors and proximal connectors (both not shown) that enable a connection to the delivery device 46. During minimally invasive surgery, the delivery device 46 transports the mitral valve 10' into the left atrium. The replacement mitral valve 10' is then placed approximate to the native mitral valve ring and deployed there. On deployment the valve is expanded from an un-deployed state into the deployed state. Thereby the plurality of anchors 22 fix one side of the atrial wall 44, whereas the plurality of fingers 18 clamp the other side of the atrial wall in order to clamp the atrial wall between the plurality of anchors 22 and the plurality of fingers 18. The delivery device 46 allows a controlled deployment at the position of the native mitral valve. It can also include additional possibilities to adjust the position of the mitral valve 10' during deployment.

In order to guide the delivery device 46 and to check the correct positioning of the mitral valve 10', this can include radiopaque markers that are preferably made of tantalum and indicate at least one of a position of the inlet flange 20, at least one of the plurality of anchors 22, at least one of a short and a long axis of an ellipse forming the mitral valve 10'.These radiopaque markers enable a tracking of the movement and position of the mitral valve 10' using e.g. x-rays.

Fig. 5 shows a further kind of stent frame 12. In this embodiment the plurality of anchors 22 only extend up to the inlet I of the body 16 of the stent frame 12. The first ends 24 of the plurality of anchors 22 are designed similar to those shown in Fig. 1 and have a generally rectangular shape.

Fig. 6a and 6b schematically show views of a further stent frame 12. The plurality of fingers 18 of this stent frame 12 do not comprise eyelets (see e.g. Fig. 1). Thereby the contact area of the plurality of fingers 18 to the anti-leakage material 28 and to the atrial wall 44 can be increased. Moreover, the anti-leakage material 28 also extends over a surface of the plurality of anchors 22 in the region of the outlet O to form an outlet flange 20'.

Tips 40' of the leaflets 40 lie in the plane of the outlet O, with the unidirectional valve member 38 dividing the valve member into the outlet O and the inlet I.

Figs. 7a & 7b schematically show views of a further kind of stent frame 12. The plurality of anchors of this stent frame 12 comprise a first group of anchors 22' and a second group of anchors 22". The first group of anchors 22' is provided for the attachment of the anti-leakage material 28 to the stent frame 12, whereas the second group of anchors 22" is provided for an attachment of the mitral valve 10' to the atrial wall 44. A respective anchor of the first group of anchors 18' is shorter than a respective anchor of the second group of anchors 18". Also a respective anchor of the first group of anchors 18' does not comprise an opening, whereas a respective anchor of the second group of anchors 22" does comprise an opening 22‴.

This design of stent frame 12 can also improve the contact area between both the anti-leakage material 28 and the stent frame 12 and the contact area between the stent frame 12 and the atrial wall 44. Moreover, one of the first and second group of anchors 22', 22" can comprise connectors (not shown) to the delivery device 46 of Fig. 4.

It should be noted in this connection that the inlet may also comprise more than one type of finger (not shown) that forms part of the inlet flange 20, with the types of fingers being arranged groupwise.

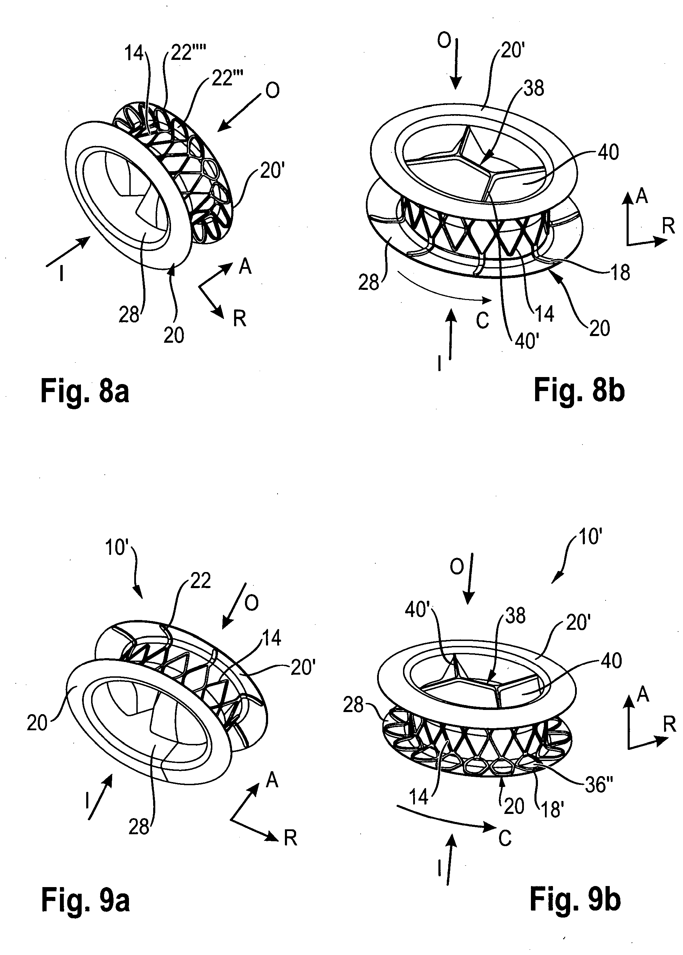

Figs. 8a & 8b schematically show views of a further stent frame 12 of this mitral valve 10'. This stent frame 12 comprises a plurality of arc shaped anchors 22"" that each comprise an opening 22". These arc shaped anchors 22"" increase the contact area between the anti-leakage material in the region of the outlet flange 20' and the stent frame 12.

Figs. 9a & 9b schematically show views of a further stent frame 12 for a mitral valve 10' which comprises a plurality of fingers 18' that are each formed of arc-shape and have an orifice 36" formed therein which hence acts as an attachment means in order to enable an improved connection of the plurality of fingers 18' to the anti-leakage material 28 and to the atrial wall 44 via the orifices 36".

The arc-shaped anchors 22' that form part of the outlet flange 20' provide an outlet flange 20' that is more rigid in comparison to the outlet flanges 20' of Figs. 6a to 8b. Such a rigid outlet flange 20' can have beneficial effects with regard to the durability of the mitral valve 10' as the pressure exerted on the mitral valve 10' is present at the outlet O and hence at the outlet flange 20'.

In this connection it should be noted that the further designs of both the plurality of fingers and the plurality of anchors can be provided at the inlet and outlet flanges respectively (not shown). The function of the plurality of fingers and of the plurality of anchors is to facilitate the connection of the valve to the atrial wall and/or to the anti-leakage material forming a seal between the different chambers of the heart.

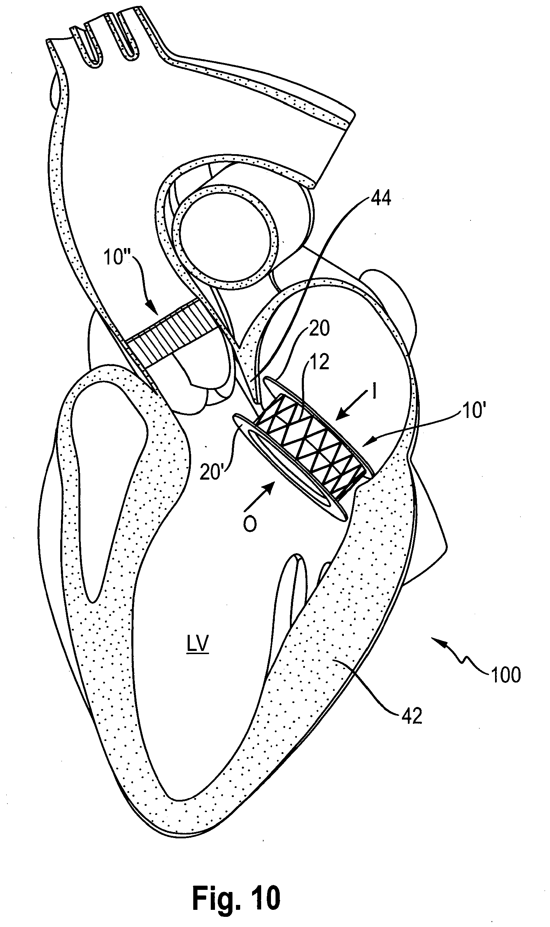

Fig. 10 shows a system of cardiac valves 100 comprising a mitral valve 10' and a replacement aortic valve 10". It has namely been found that once e.g. a mitral valve 10' has been positioned in the heart 42, the outflow properties of the mitral valve 10' are significantly enhanced in comparison to the defective native mitral valve and the downstream native aortic valve may have become accustomed to the behavior of the native mitral valve such that its performance needs enhancing following mitral valve replacement surgery.

### List of reference numerals:

- 10: atrioventricular valve
- 10': mitral valve
- 10": aortic valve
- 12: stent frame
- 14: elongate struts
- 16: body
- 18, 18': finger
- 20: inlet flange
- 20': outlet flange
- 22, 22ʺʺ: anchor
- 22', 22": groups of anchors
- 22‴: anchor opening
- 24: first end
- 26: second end
- 28: anti-leakage material
- 30: inner surface of body
- 32: outer surface of finger
- 32': outer surface of body
- 34: suture
- 36: eyelet
- 36': aperture
- 36": orifice of arc shaped finger 18'
- 38: valve member
- 40: leaflet
- 40': tip of leaflet
- 42: heart
- 44: atrial wall
- 46: delivery device
- 100: system of cardiac valves

- A: axial direction
- B: radial direction
- C: circumferential direction
- Dᵢ: inner diameter of the body at the inlet
- Dₒ: inner diameter of the body at the outlet
- D: inner diameter of the body
- H: height
- H_{b}: height of body
- I: inlet
- L: lower end
- LV: left ventricle
- O: outlet
- RA: right atrium
- RV: right ventricle
- U: upper end

## Claims

1. A self-expandable atrioventricular valve (10, 10') that is expandable from an un-deployed state into a deployed state and that in the deployed state comprises:
- a stent frame (12) forming a body (16), with the body (16) having a height (H_{b}) extending from an inlet (I) to an outlet (O) of the self-expandable atrioventricular valve (10, 10') in an axial direction (A) and with the body (16) circumferentially extending in a circumferential direction (C) around the axial direction (A),
- a unidirectional valve member (38) arranged within the body (16) in the region of the outlet (O),
- a plurality of fingers (18; 18') that is arranged in the region of the inlet (I), with the plurality of fingers (18; 18') projecting radially outwardly from the body (16) in a radial direction (B) and with the plurality of fingers (18; 18') forming at least part of an upper end (U) of the self-expandable atrioventricular valve (10, 10') together with the inlet (I),
- a plurality of anchors (22; 22', 22") that is arranged in the region of the outlet (O), with a first end (24) of each of the plurality of anchors (22; 22', 22") projecting radially outwardly from the body (16) in the radial direction (B) in the region of the outlet (O) and with the first ends (24) of the plurality of anchors (22; 22', 22") forming at least part of a lower end (L) of the self-expandable atrioventricular valve (10, 10') together with the outlet (O);
**characterised in that**
at least some of the plurality of anchors (22) is configured as respective commissures for the valve member (38) that is longitudinally attached to the plurality of anchors (22) over the height (H_{b}) of the body (16) and circumferentially within the body (16) in the region of the inlet (I), so that an upper surface of the valve member (38) is present in the region of the inlet (I) and preferably forms part of the upper end (U); and
further comprising a plurality of elongate struts (14) forming at least part of the body (16) of the stent frame (12), wherein the plurality of elongate struts (14), the plurality of fingers (18; 18') and the plurality of anchors (22; 22', 22") are formed from the same material, such as nitinol, have at least approximately the same thickness in the radial direction (B), and with the plurality of anchors (22; 22', 22") and the plurality of fingers (18; 18') respectively having a greater width than the plurality of elongate struts (14) in the circumferential direction (C) of the body (16).

2. A self-expandable atrioventricular valve in accordance with claim 1, wherein the stent frame (12) at least substantially has the shape of an inner bearing race.

3. A self-expandable atrioventricular valve in accordance with claim 1 or claim 2, wherein the height (H_{b}) of the body (16) is selected in the range of 0.3 to 2.0 cm; and/or wherein an overall height (H) of the atrioventricular valve (10, 10') between an uppermost point of the upper end (U) and a lowermost point of the lower end (L) is selected in the range of 0.3 to 2.5 cm.

4. A self-expandable atrioventricular valve in accordance with any one of the preceding claims, wherein the valve member (38) is longitudinally attached to second ends (26) of the plurality of anchors (22).

5. A self-expandable atrioventricular valve in accordance with any one of the preceding claims, wherein the plurality of anchors (22; 22', 22") radially project away from the outlet (O) towards the upper end (U); and/or wherein the plurality of fingers (18; 18') and the plurality of anchors (22; 22', 22') are arranged such that they at least generally project in the same axial direction (A).

6. A self-expandable atrioventricular valve in accordance with any one of the preceding claims, wherein a second end (26) of the plurality of anchors (22) extends over the height (H_{b}) of the body (16) in the axial direction (A)..

7. A self-expandable atrioventricular valve in accordance with any one of the preceding claims, wherein the valve member (38) comprises at least two leaflets (40), and preferably comprises four leaflets (40).

8. A self-expandable atrioventricular valve in accordance with any one of the preceding claims, wherein the plurality of fingers (18; 18') form a part of an inlet flange (20) of the self-expandable atrioventricular valve (10, 10') forming the upper end (U) and/or wherein the plurality of anchors (22; 22', 22") form a part of an outlet flange (20') of the self-expandable atrioventricular valve (10, 10') forming the lower end (L).

9. A self-expandable atrioventricular valve in accordance with any one of the preceding claims, wherein the plurality of fingers (18; 18') and the plurality of anchors (22; 22', 22") act as cooperating clamps of the self-expandable atrioventricular valve (10, 10').

10. A self-expandable atrioventricular valve in accordance with any one of the preceding claims, wherein the plurality of anchors (22; 22', 22") is formed by or extends from at least some of the material forming the body (16); and/or wherein the plurality of anchors comprise a first group of anchors (22') and a second group of anchors (22"), with a respective anchor of the first group of anchors (22') being shorter than a respective anchor of the second group of anchors (22"); and/or wherein the plurality of fingers (18; 18') is formed by or extends from at least some of the material forming the body (16); and/or wherein the plurality of fingers comprise a first group of fingers and a second group of fingers, with a respective finger of the first group of fingers being shorter than a respective finger of the second group of fingers.

11. A self-expandable atrioventricular valve in accordance with any one of the preceding claims, wherein the body (16) has an at least generally elliptical shape in a cross-section perpendicular to the axial direction (A).

12. A self-expandable atrioventricular valve in accordance with any one of the preceding claims, wherein the body (16) has an at least generally cylindrical outer shape over its height (H_{b}) in the axial direction (A).

13. A self-expandable atrioventricular valve in accordance with any one of the preceding claims, wherein a diameter (Dᵢ) of the body (16) at the inlet (I) is greater than or equal to a diameter (Dₒ) of the body (16) at the outlet (O) in a cross-section perpendicular to the axial direction (A).

14. A self-expandable atrioventricular valve in accordance with any one of the preceding claims, further comprising anti-leakage material (28), with the anti-leakage material being arranged to cover at least part of an inner and/or an outer surface (30, 32') of the body (14), preferably with the anti-leakage material (28) being arranged to at least substantially completely cover the inner and/or the outer surface (30, 32') of the body (14), optionally wherein the anti-leakage material (28) at least partly covers the plurality of fingers (18; 18') that form the upper end (U) and/or the plurality of anchors (22; 22', 22") that form the lower end (L).

15. A system (100) of cardiac valves comprising a self-expandable atrioventricular valve (10, 10') in accordance with at least one of the preceding claims, and a surgical valve (10") for replacement of one of the aortic valve and the pulmonary valve.

## Patentansprüche

1. Selbstexpandierbare Atrioventrikularklappe (10, 10'), die von einem nicht entfalteten Zustand in einen entfalteten Zustand expandierbar ist und die in dem entfalteten Zustand umfasst:
- einen Stentrahmen (12), der einen Körper (16) bildet, wobei der Körper (16) eine Höhe (H_{b}) aufweist, die sich von einem Einlass (I) zu einem Auslass (O) der selbstexpandierbaren Atrioventrikularklappe (10, 10') in einer axialen Richtung (A) erstreckt, und wobei sich der Körper (16) in einer Umfangsrichtung (C) um die axiale Richtung (A) herum erstreckt,
- ein unidirektionales Klappenelement (38), das innerhalb des Körpers (16) im Bereich des Auslasses (O) angeordnet ist,
- eine Vielzahl von Fingern (18; 18'), die im Bereich des Einlasses (I) angeordnet ist, wobei die Vielzahl von Fingern (18; 18') von dem Körper (16) in einer radialen Richtung (B) radial nach außen ragt und wobei die Vielzahl von Fingern (18; 18') zusammen mit dem Einlass (I) zumindest einen Teil eines oberen Endes (U) der selbstexpandierbaren Atrioventrikularklappe (10, 10') bildet,
- eine Vielzahl von Ankern (22; 22', 22"), die im Bereich des Auslasses (O) angeordnet ist, wobei ein erstes Ende (24) jedes der Vielzahl von Ankern (22; 22', 22") im Bereich des Auslasses (O) vom Körper (16) in radialer Richtung (B) radial nach außen ragt und wobei die ersten Enden (24) der Vielzahl von Ankern (22; 22', 22") zusammen mit dem Auslass (O) zumindest einen Teil eines unteren Endes (L) der selbstexpandierbaren Atrioventrikularklappe (10, 10') bilden;
**dadurch gekennzeichnet, dass**
zumindest ein Teil der Vielzahl von Ankern (22) jeweils als Kommissuren für das Klappenelement (38) ausgebildet ist, das über die Höhe (H_{b}) des Körpers (16) und in Umfangsrichtung innerhalb des Körpers (16) im Bereich des Einlasses (I) an der Vielzahl von Ankern (22) in Längsrichtung befestigt ist, so dass eine obere Fläche des Klappenelements (38) im Bereich des Einlasses (I) vorhanden ist und vorzugsweise einen Teil des oberen Endes (U) bildet; und
ferner mit einer Vielzahl von länglichen Streben (14), die zumindest einen Teil des Körpers (16) des Stentrahmens (12) bilden, wobei die Vielzahl von länglichen Streben (14), die Vielzahl von Fingern (18; 18') und die Vielzahl von Ankern (22; 22', 22") aus demselben Material, wie Nitinol, gebildet sind, zumindest annähernd dieselbe Dicke in der radialen Richtung (B) haben und wobei die Vielzahl von Ankern (22; 22', 22") und die Vielzahl von Fingern (18; 18') jeweils eine größere Breite als die Vielzahl von länglichen Streben (14) in der Umfangsrichtung (C) des Körpers (16) haben.

2. Selbstexpandierbare Atrioventrikularklappe nach Anspruch 1, wobei der Stentrahmen (12) zumindest im Wesentlichen die Form einer inneren Lagerbahn aufweist.

3. Selbstexpandierbare Atrioventrikularklappe nach einem der Ansprüche 1 oder 2, wobei die Höhe (H_{b}) des Körpers (16) im Bereich von 0,3 bis 2,0 cm gewählt ist; und/oder wobei eine Gesamthöhe (H) der Atrioventrikularklappe (10, 10') zwischen einem obersten Punkt des oberen Endes (U) und einem untersten Punkt des unteren Endes (L) im Bereich von 0,3 bis 2,5 cm gewählt ist.

4. Selbstexpandierbare Atrioventrikularklappe nach einem der vorhergehenden Ansprüche, wobei das Klappenelement (38) in Längsrichtung an zweiten Enden (26) der Vielzahl von Ankern (22) befestigt ist.

5. Selbstexpandierbare Atrioventrikularklappe nach einem der vorhergehenden Ansprüche, wobei die Vielzahl der Anker (22; 22', 22") radial vom Auslass (O) zum oberen Ende (U) weg vorragen; und/oder wobei die Vielzahl von Fingern (18; 18') und die Vielzahl von Ankern (22; 22', 22") so angeordnet sind, dass sie zumindest im Wesentlichen in dieselbe axiale Richtung (A) ragen.

6. Selbstexpandierbare Atrioventrikularklappe nach einem der vorhergehenden Ansprüche, wobei sich ein zweites Ende (26) der Vielzahl von Ankern (22) über die Höhe (H_{b}) des Körpers (16) in axialer Richtung (A) erstreckt.

7. Selbstexpandierbare Atrioventrikularklappe nach einem der vorhergehenden Ansprüche, wobei das Klappenelement (38) mindestens zwei Segel (40) und vorzugsweise vier Segel (40) aufweist.

8. Selbstexpandierbare Atrioventrikularklappe nach einem der vorhergehenden Ansprüche, wobei die Vielzahl der Finger (18; 18') einen Teil eines Einlassflansches (20) der selbstexpandierbaren Atrioventrikularklappe (10, 10') bildet, der das obere Ende (U) bildet, und/oder wobei die Vielzahl von Ankern (22; 22', 22") einen Teil eines Auslassflansches (20') der selbstexpandierbaren Atrioventrikularklappe (10, 10') bildet, der das untere Ende (L) bildet.

9. Selbstexpandierbare Atrioventrikularklappe nach einem der vorhergehenden Ansprüche, wobei die Vielzahl von Fingern (18; 18') und die Vielzahl von Ankern (22; 22', 22") als zusammenwirkende Klammern der selbstexpandierbaren Atrioventrikularklappe (10, 10') wirken.

10. Selbstexpandierbare Atrioventrikularklappe nach einem der vorhergehenden Ansprüche, wobei die Vielzahl von Ankern (22; 22', 22") durch mindestens einen Teil des den Körper (16) bildenden Materials gebildet ist oder sich von diesem erstreckt; und/oder wobei die mehreren Anker eine erste Gruppe von Ankern (22') und eine zweite Gruppe von Ankern (22") umfassen, wobei ein entsprechender Anker der ersten Gruppe von Ankern (22') kürzer ist als ein entsprechender Anker der zweiten Gruppe von Ankern (22"); und/oder wobei die Vielzahl von Fingern (18; 18') aus mindestens einem Teil des den Körper (16) bildenden Materials gebildet ist oder sich von diesem erstreckt; und/oder wobei die Vielzahl von Fingern eine erste Gruppe von Fingern und eine zweite Gruppe von Fingern umfasst, wobei ein entsprechender Finger der ersten Gruppe von Fingern kürzer ist als ein entsprechender Finger der zweiten Gruppe von Fingern.

11. Selbstexpandierbare Atrioventrikularklappe nach einem der vorhergehenden Ansprüche, wobei der Körper (16) in einem Querschnitt senkrecht zur axialen Richtung (A) eine zumindest allgemein elliptische Form aufweist.

12. Selbstexpandierbare Atrioventrikularklappe nach einem der vorhergehenden Ansprüche, wobei der Körper (16) über seine Höhe (H_{b}) in axialer Richtung (A) eine zumindest im Wesentlichen zylindrische Außenform aufweist.

13. Selbstexpandierbare Atrioventrikularklappe nach einem der vorhergehenden Ansprüche, wobei ein Durchmesser (Dᵢ) des Körpers (16) am Einlass (I) größer oder gleich einem Durchmesser (Dₒ) des Körpers (16) am Auslass (O) in einem Querschnitt senkrecht zur axialen Richtung (A) ist.

14. Selbstexpandierbare Atrioventrikularklappe nach einem der vorhergehenden Ansprüche, ferner umfassend ein Anti-Leck-Material (28), wobei das Anti-Leck-Material so angeordnet ist, dass es zumindest einen Teil einer inneren und/oder einer äußeren Fläche (30, 32') des Körpers (14) bedeckt, wobei das Anti-Leck-Material (28) vorzugsweise so angeordnet ist, dass es die innere und/oder die äußere Fläche (30, 32') des Körpers (14) zumindest im Wesentlichen vollständig bedeckt, wobei das Anti-Leck-Material (28) optional zumindest teilweise die Vielzahl von Fingern (18; 18'), die das obere Ende (U) bildet, und/oder die Vielzahl von Ankern (22; 22', 22"), die das untere Ende (L) bildet, bedeckt.

15. System (100) aus Herzklappen, umfassend eine selbstexpandierbare Atrioventrikularklappe (10, 10') nach einem der vorhergehenden Ansprüche und eine chirurgische Klappe (10") zum Austausch der Aortenklappe oder der Pulmonalklappe.

## Revendications

1. Valvule atrioventriculaire auto-extensible (10, 10') qui est extensible depuis un état non déployé jusqu'à un état déployé et qui, dans l'état déployé, comprend :
- un châssis d'endoprothèse (12) formant un corps (16), le corps (16) ayant une hauteur (H_{b}) s'étendant depuis une entrée (I) jusqu'à une sortie (O) de la valvule atrioventriculaire auto-extensible (10, 10') dans une direction axiale (A) et le corps (16) s'étendant circonférentiellement dans une direction circonférentielle (C) autour de la direction axiale (A),
- un élément valvulaire multidirectionnel (38) agencé à l'intérieur du corps (16) dans la région de la sortie (O),
- une pluralité de doigts (18 ; 18') qui sont agencés dans la région de l'entrée (I), la pluralité de doigts (18 ; 18') se projetant radialement vers l'extérieur depuis le corps (16) dans une direction radiale (B) et la pluralité de doigts (18 ; 18') formant au moins une partie d'une extrémité supérieure (U) de la valvule atrioventriculaire auto-extensible (10, 10') conjointement avec l'entrée (I),
- une pluralité d'ancrages (22 ; 22', 22") qui sont agencés dans la région de la sortie (O), une première extrémité (24) de chacun de la pluralité d'ancrages (22 ; 22', 22") se projetant radialement vers l'extérieur depuis le corps (16) dans la direction radiale (B) dans la région de la sortie (O), et les premières extrémités (24) de la pluralité d'ancrages (22 ; 22', 22") formant au moins une partie d'une extrémité inférieure (L) de la valve atrioventriculaire auto-extensible (10, 10') conjointement avec la sortie (O) ;
**caractérisée en ce que**
au moins certains de la pluralité d'ancrages (22) sont configurés comme commissures respectives pour l'élément valvulaire (38) qui est longitudinalement fixé à la pluralité d'ancrages (22) sur la hauteur (H_{b}) du corps (16) et circonférentiellement à l'intérieur du corps (16) dans la région de l'entrée (I), de telle sorte qu'une surface supérieure de l'élément valvulaire (38) est présente dans la région de l'entrée (I) et forme de préférence une partie de l'extrémité supérieure (U) ; et
comprenant en outre une pluralité de tiges allongées (14) formant au moins une partie du corps (16) du châssis d'endoprothèse (12), dans laquelle la pluralité de tiges allongées (14), la pluralité de doigts (18 ; 18') et la pluralité d'ancrages (22 ; 22', 22") sont formés à partir du même matériau, comme du Nitinol, et ont au moins approximativement la même épaisseur dans la direction radiale (B), et la pluralité d'ancrages (22 ; 22', 22") et la pluralité de doigts (18 ; 18') ayant respectivement une largeur supérieure à la pluralité de tiges allongées (14) dans la direction circonférentielle (C) du corps (16).

2. Valvule atrioventriculaire auto-extensible selon la revendication 1, dans laquelle le châssis d'endoprothèse (12) a au moins sensiblement la forme d'une piste de palier intérieure.

3. Valvule atrioventriculaire auto-extensible selon la revendication 1 ou 2, dans laquelle la hauteur (H_{b}) du corps (16) est sélectionnée dans la plage de 0,3 à 2,0 cm ; et/ou dans laquelle une hauteur totale (H) de la valvule atrioventriculaire (10, 10') entre un point le plus supérieur de l'extrémité supérieure (U) et un point le plus inférieur de l'extrémité inférieure (L) est sélectionnée dans la plage de 0,3 à 2,5 cm.

4. Valvule atrioventriculaire auto-extensible selon l'une quelconque des revendications précédentes, dans laquelle l'élément valvulaire (38) est longitudinalement fixé à des secondes extrémités (26) de la pluralité d'ancrages (22).

5. Valvule atrioventriculaire auto-extensible selon l'une quelconque des revendications précédentes, dans laquelle la pluralité d'ancrages (22 ; 22', 22") se projettent radialement en éloignement de la sortie (O) vers l'extrémité supérieure (U) ; et/ou dans laquelle la pluralité de doigts (18 ; 18') et la pluralité d'ancrages (22 ; 22', 22") sont agencés de telle sorte qu'il se projette au moins généralement dans la même direction axiale (A).

6. Valvule atrioventriculaire auto-extensible selon l'une quelconque des revendications précédentes, dans laquelle une seconde extrémité (26) de la pluralité d'ancrages (22) s'étend sur la hauteur (H_{b}) du corps (16) dans la direction axiale (A).

7. Valvule atrioventriculaire auto-extensible selon l'une quelconque des revendications précédentes, dans laquelle l'élément valvulaire (38) comprend au moins deux lames (40), et de préférence comprend quatre lames (40).

8. Valvule atrioventriculaire auto-extensible selon l'une quelconque des revendications précédentes, dans laquelle la pluralité de doigts (18 ; 18') forment une partie d'une bride d'entrée (20) de la valvule atrioventriculaire auto-extensible (10, 10') formant l'extrémité supérieure (U) et/ou dans laquelle la pluralité d'ancrages (22 ; 22', 22") forment une partie d'une bride de sortie (20') de la valvule atrioventriculaire auto-extensible (10, 10') formant l'extrémité inférieure (L).

9. Valvule atrioventriculaire auto-extensible selon l'une quelconque des revendications précédentes, dans laquelle la pluralité de doigts (18 ; 18') et la pluralité d'ancrages (22 ; 22', 22") agissent en tant que pinces coopérantes de la valvule atrioventriculaire auto-extensible (10, 10').

10. Valvule atrioventriculaire auto-extensible selon l'une quelconque des revendications précédentes, dans laquelle la pluralité d'ancrages (22 ; 22', 22") sont formés par ou s'étendent depuis au moins un certain matériau formant le corps (16) ; et/ou dans laquelle la pluralité d'ancrages comprennent un premier groupe d'ancrages (22') et un second groupe d'ancrages (22"), un ancrage respectif du premier groupe d'ancrages (22') étant plus court qu'un ancrage respectif du second groupe d'ancrages (22") ; et/ou dans laquelle la pluralité de doigts (18 ; 18') sont formés par ou s'étendent depuis au moins un certain matériau formant le corps (16) ; et/ou dans laquelle la pluralité de doigts comprennent un premier groupe de doigts et un second groupe de doigts, un doigt respectif du premier groupe de doigts étant plus court qu'un doigt respectif du second groupe de doigts.

11. Valvule atrioventriculaire auto-extensible selon l'une quelconque des revendications précédentes, dans laquelle le corps (16) a une forme au moins généralement elliptique dans une section transversale perpendiculaire à la direction axiale (A).

12. Valvule atrioventriculaire auto-extensible selon l'une quelconque des revendications précédentes, dans laquelle le corps (16) a une forme extérieure au moins généralement cylindrique sur sa hauteur (H_{b}) dans la direction axiale (A).

13. Valvule atrioventriculaire auto-extensible selon l'une quelconque des revendications précédentes, dans laquelle un diamètre (Dᵢ) du corps (16) au niveau de l'entrée (I) est supérieur ou égal à un diamètre (Dₒ) du corps (16) au niveau de la sortie (O) dans une section transversale perpendiculaire à la direction axiale (A).

14. Valvule atrioventriculaire auto-extensible selon l'une quelconque des revendications précédentes, comprenant en outre un matériau anti-fuite (28), le matériau anti-fuite étant agencé pour couvrir au moins une partie d'une surface intérieure et/ou extérieure (30, 32') du corps (16), de préférence le matériau anti-fuite (28) étant agencé pour couvrir au moins sensiblement complètement la surface intérieure et/ou extérieure (30, 32') du corps(16), en option dans laquelle le matériau anti-fuite (28) couvre au moins partiellement la pluralité de doigts (18 ; 18') qui forment l'extrémité supérieure (U) et/ou la pluralité d'ancrages (22 ; 22', 22") qui forment l'extrémité inférieure (L).

15. Système (100) valvulaire cardiaque comprenant une valvule atrioventriculaire extensible (10, 10') selon l'une au moins des revendications précédentes, et une valvule chirurgicale (10") destinée à remplacer une valvule parmi la valvule aortique et la valvule pulmonaire.
